# EUROPEAN PATENT APPLICATION

(11) **EP 2 055 309 A1**
(43) Date of publication of application: **06.05.2009**
(21) Application number: 07119772.7
(22) Date of filing: 31.10.2007
(51) Int. Cl.: A61K 38/14, A61K 47/20

(54) **Vancomycin and Teicoplanin anhydrous formulations for topical use**

(71) Applicant: Pharmatex Italia Srl, 20121 Milano (IT)
(72) Inventor: De Tommaso, Vincenzo, Dr., 20052, Monza (IT)
(74) Representative: Serravalle, Marco

(57) **Abstract**

The invention concerns pharmaceutical anhydrous formulation for topical use comprising Vancomycin, Vancomycin Hydrochloride, or Teicoplanin, and Dimethylsulfoxide.

In a preferred embodiment the formulation further comprises one or more glycols and/or ethers thereof, and optionally one or more fatty acids triglycerides and/or the polyoxyethylene derivative thereof.

The use of Dimethylsulfoxide in anhydrous formulation for topical use leads to formulations characterized by high homogeneity and high concentration, if desired, of Vancomycin, Vancomycin Hydrochloride, or Teicoplanin. These Dimethylsulfoxide-containing formulations are also very stable in time.

## Description

The present invention concerns anhydrous formulations based on Teicoplanin, Vancomycin and Vancomycin Hydrochloride for topical use.

Vancomycin, Vancomycin Hydrochloride and Teicoplanin are glycopeptide antibiotics having a broad spectrum of activity.

Vancomycin and Vancomycin Hydrochloride are produced by strains of species Mycropolyspora orientalis, and isolated from the fermentation broth in which it has been produced. These substances are useful in the treatment in the form of the free base or in the form of hydrochloride. Vancomycin and Vancomycin Hydrochloride act by inhibiting proper cell wall synthesis in Gram-positive bacteria.

Teicoplanin is an antibiotic used in the prophylaxis and treatment of serious infections caused by Gram-positive bacteria, including methicillin-resistant Staphylococcus aureus and Enterococcus faecalis. It is a glycopeptide antiobiotic extracted from Actinoplanes teichomyceticus, with a similar spectrum of activity to Vancomycin.

Teicoplanin and Vancomycin Hydrochloride are very soluble in water and poorly soluble in organic solvents. This fact makes it difficult to prepare anhydrous pharmaceutical compositions for topical use and, up till now, there are no topical formulations of Vancomycin Hydrochloride and Teicoplanin wherein the antibiotic is present at high concentration and homogeneously dispersed.

WO 02/04012 discloses anhydrous pharmaceutical compositions for topical use comprising Vancomycin and Vancomycin Hydrochloride, one or more glycols and/or ethers thereof, one or more fatty acids triglycerides and/or the polyoxyethylene derivative thereof and a gelling agent. However, these formulations allow solubilization of only a limited amount of Vancomycin Hydrochloride.

It has been surprisingly found that the use of Dimethylsulfoxide in anhydrous formulation for topical use leads to formulations characterized by high homogeneity and high concentration, if desired, of Vancomycin, Vancomycin Hydrochloride, or Teicoplanin. These Dimethylsulfoxide-containing formulations are also very stable in time.

The present invention provides homogeneous and stable anhydrous pharmaceutical formulations comprising:
a) Vancomycin, Vancomycin Hydrochloride, or Teicoplanin,
b) Dimethylsulfoxide.

In a preferred embodiment of the invention the formulation further comprises:
c) one or more glycols and/or ethers thereof,
d) optionally one or more fatty acids triglycerides and/or the polyoxyethylene derivative thereof.

Vancomycin, Vancomycin Hydrochloride, or Teicoplanin are preferably present in an amount varying from 0.1 to 20 % by weight of the total composition, preferably from 0.5 to 15 %. In the case of Vancomycin Hydrochloride, the amount is most preferably between 2 and 12 % by weight of the total composition. In the case of Teicoplanin the amount is most preferably comprised between 0.5 and 5 % by weight of the total composition.

The presence of Dimethylsulfoxide (DMSO, component b)) is essential to obtain a homogeneous and stable formulation containing, if desired, a high concentration of antibiotic. Water is an excellent solvent for Vancomycin Hydrochloride and Teicoplanin but, at the same time, it favours their decomposition; on the contrary, DMSO possesses excellent solvent properties but does not promote decomposition of these compounds. Preferably Dimethylsulfoxide is present in an amount comprised between 1 and 80 % by weight of the total composition, more preferably between 5 and 50 % by weight, most preferably between 8 and 30 % by weight of the total composition.

The glycols and/or ethers thereof are preferably ethylene glycol, propylene glycol, diethylene glycol monomethyl ether, diethylene glycol monoethyl ether and combinations thereof. They are preferably present in an amount comprised between 10 and 95 % by weight of the total composition, more preferably comprised between 20 and 90 %, most preferably between 30 and 85 %.

The fatty acid triglycerides and/or polyoxyethylene derivatives thereof, when present, are preferably chosen from the group consisting of C₈, C₁₀, C₁₂, C₁₄, C₁₆, C₁₈, C₂₀ fatty acids triglycerides and/or the polyoxyethylene derivatives thereof wherein the polyoxyethylene moiety has preferably a molecular weight from 200 to 10,000 Da. Labrasol (polyethylene glycol C₈₋₁₀ glycerides) is particularly preferred. Component c) is preferably present in an amount comprised between 0 and 30 % by weight of the total composition, more preferably comprised between 0 and 25 %, most preferably between 0 and 20 %.

The pharmaceutical formulation of the present invention is preferably in the form of a gel or a solution.

When the formulation is a gel, it further comprises a gelling agent. The gelling agent is preferably a cellulose ester or ether, a (co)polymer of (meth)acrylic acid or ester, xanthan gum, carrageenin. A preferred gelling agent is Carbopol ^{™}, which is a polymer of acrylic acid, crosslinked with allyl ethers of sucrose or pentaerythritol. The gelling agent is preferably present in an amount comprised between 0.1 and 20 % by weight of the total composition, more preferably comprised between 0.5 and 15 %, most preferably between 0.5 and 5 %.

The formulation of the present invention can further include other ingredients commonly used in topical formulations, e.g. surfactants and emulsifiers.

Surfactants for use in the present invention are preferably non-ionic, cationic and anionic. A preferred non-ionic surfactant is polyoxyethylene stearyl ether. Preferred cationic surfactants are quaternary ammonium salts. A preferred anionic surfactant is sodium lauryl sulphate.

### Examples

### Preparation of gel of Vancomycin Hydrochloride and Teicoplanin

The antibiotic was dissolved in DMSO. Propylenglycol and Transcutol P^{™} were added in this order to the solution under stirring and the mixing continued for 10 minutes after addition.

Carbopol^{™} was added to the solution and the mixture was mixed until formation of a gel. The gel was left to rest for at least 18 h and then stirred vigorously for at least 1 h.

### Example 1

### 3% Vancomycin gel

Composition for 100 g:

| | | |
|---|---|---|
| • | Vancomycin Hydrochloride | 3 g |
| • | Dimethylsulfoxide | 14 g |
| • | Propylenglycol | 70.4 g |
| • | Transcutol P^{™} (Diethylene Glycol Monoethyl Ether) | 10 g |
| • | Carbopol^{™} | 2.6 g |

### Example 2

### 5% Vancomycin gel

Composition for 100 g:

| | | |
|---|---|---|
| • | Vancomycin Hydrochloride | 5 g |
| • | Dimethylsulfoxide | 18 g |
| • | Propylenglycol | 67 g |
| • | Transcutol P^{™} (Diethylene Glycol Monoethyl Ether) | 8 g |
| • | Carbopol^{™} | 2 g |

### Example 3

### 3% Teicoplanin gel

Composition for 100 g:

| | | |
|---|---|---|
| • | Teicoplanin | 3 g |
| • | Dimethylsulfoxide | 14 g |
| • | Propylenglycol | 70.85 g |
| • | Transcutol P^{™} (Diethylene Glycol Monoethyl Ether) | 10 g |
| • | Carbopol^{™} | 2.15 g |

### Example 4

### 1% Teicoplanin gel

Composition for 100 g:

| | | |
|---|---|---|
| • | Teicoplanin | 1 g |
| • | Dimethylsulfoxide | 14 g |
| • | Propylenglycol | 72.7 g |
| • | Transcutol P^{™} (Diethylene Glycol Monoethyl Ether) | 10 g |
| • | Carbopol^{™} | 2.3 g |

### Example 5

### 10% Vancomycin solution

Composition for 100 g:

| | | |
|---|---|---|
| • | Vancomycin Hydrochloride | 10 g |
| • | Dimethylsulfoxide | 25 g |
| • | Propylenglycol | 64.7 g |
| • | Transcutol P^{™} (Diethylene Glycol Monoethyl Ether) | 0.3 g |

Vancomycin Hydrochloride was dissolved in DMSO. Propylenglycol and Transcutol P^{™} were added in this order to the solution under stirring and the mixing continued for 15 minutes after addition. The obtained solution was clear indicating that Vancomycin was completely dissolved.

## Claims

1. Pharmaceutical anhydrous formulation for topical use comprising:
a. Vancomycin, Vancomycin Hydrochloride, or Teicoplanin,
b. Dimethylsulfoxide.

2. Formulation according to claim 1 further comprising:
c. one or more glycols and/or ethers thereof,
d. optionally one or more fatty acids triglycerides and/or the polyoxyethylene derivative thereof.

3. Formulation according to claims 1-2 wherein dimethylsulfoxide is present in an amount comprised from 1 and 80 % by weight of the total composition.

4. Formulations according to claims 1-3 wherein component c) is selected from ethylene glycol, propylene glycol, diethylene glycol monomethyl ether, diethylene glycol monoethyl ether and combinations thereof.

5. Formulation according to claims 1-4 wherein the formulation is in the form of a gel and further comprises a gelling agent

6. Gel according to claim 5 wherein the gelling agent is selected from a cellulose ester or ether, a (co)polymer of (meth)acrylic acid or ester, xanthan gum, carrageenin.

7. Formulation according to claims 1-4 wherein the formulation is in the form of a solution.

8. Formulation according to claims 1-7 wherein the amount of dimethylsulfoxide is comprised between 5 and 50 %, preferably between 8 and 30 % by weight of the total composition.

9. Formulation according to claims 1-8 wherein the formulation comprises from 2 to 12 % by weight of Vancomycin Hydrochloride.

10. Formulation according to claims 1-8 wherein the formulation comprises from 0.5 to 5 % by weight of Teicoplanin.
